# EUROPEAN PATENT SPECIFICATION

(11) **EP 1 442 753 B1**
(45) Date of publication and mention of the grant of the patent: **21.02.2007**
(21) Application number: 03002292.5
(22) Date of filing: 03.02.2003
(51) Int. Cl.: A61L 2/16, A61L 2/18, A61L 29/16, A61L 33/00, A61L 31/14, A61L 31/16, A61M 25/00

(54) **Composition for the prevention of indwelling device related infection**
Zusammensetzung zur Prävention von Infektionen durch subkutane Prothesen
Composition pour la prévention des infections par prothèses endovasculaires

(43) Date of publication of application: 04.08.2004
(73) Proprietor: Polaschegg, Hans-Dietrich, Dr.techn., 9231 Köstenberg (AT)
(72) Inventor: Polaschegg, Hans-Dietrich, Dr.techn., 9231 Köstenberg (AT)
(74) Representative: Kunz, Herbert

(56) References cited:
- EP-A- 1 040 841
- EP-A- 1 103 251
- WO-A-94/03174
- WO-A-98/28027
- US-A- 4 587 284
- US-A- 4 905 700
- US-A- 6 166 007
- US-A1- 2002 010 438
- US-B1- 6 451 003

## Description

### Field of the Invention

This invention relates to methods and compositions, which prevent biofilm formation on or near medical prosthetic devices in order to reduce patient infection related to indwelling devices. The invention further addresses the complete life cycle of medical applications of an indwelling device teaching means to reduce or prevent infection.

### Background

Medical prosthetic devices are widely used in medicine to treat chronic medical conditions as well as providing acute medical care. It is well known that implanted medical devices are often colonized with microbes and patients with medical devices have higher risk of becoming infected than patients without implanted devices. A study published in 1995 examined 38 ex vivo catheters from patients receiving chemotherapy. All catheters were coated with Biofilm even those implanted for only a few days and even in patients without signs of infection had colonized catheters (Anaissie E, Samonis G, Kontoyiannis D, Costerton J, Sabharwal U, Bodey G, Raad I. Role of catheter colonization and infrequent hematogenous seeding in catheter-related infections.. Eur J Clin Microbiol Infect Dis 1995;14:134-7) A German study on silver impregnated catheters showed that bacteria could be found on tips of catheters explanted after only 6 days of use. The likelihood for finding colonized catheter tips increased with the number of days of use (Boswald M, Lugauer S, Regenfus A, Braun GG, Martus P, Geis C, Scharf J, Bechert T, Greil J, Guggenbichler JP; Reduced rates of catheter-associated infection by use of a new silver-impregnated central venous catheter. Infection 1999;27:S56-60, German version: Infection 1998;26:S61-6)

A new sub-study branch has developed in Microbiology to study Biofilm (Costerton JW, Stewart PS, Greenberg EP. Bacterial Biofilms: A Common Cause of Persistent Infections. Science 1999;284:1318-22) The Biofilm specialty has adopted new methods of imaging and quantification techniques to study this form of microbial populations. These new techniques helped elucidate the sequencing steps of Biofilm formation and show how most infections in patients with indwelling medical devices can be traced to the Biofilm adhering to the device. In the last years scientists have discovered that microbes in a Biofilm are considerably different from the microbes in the planktonic form, which were the only microbes studied previously.

Many hospitalized patients often become infected while in the hospital for unrelated diseases. Hospital acquired infections are called nosocomial infections and the incidence of these infections has been growing rapidly in the advanced countries of the world. The increase is caused by several factors including the emergence of bacteria resistant to antibiotics and the increased use of invasive medical devices and procedures. Catheters account for approximately 50% of all nosocomial bloodstream infections resulting in ~400,000 episodes of catheter related bloodstream infections (CRBSI) each year in the USA. These increase the average length of stay in the intensive care unit (ICU) by 20 days with a medium cost of approximately US$ 92,000 per patient and is the cause of death of 15 to 35% of patients in the ICU. (Ryder M., The Role of Biofilm in Vascular Catheter Related Infection. New Developments in Vascular Disease published by Physicians & Scientists Publishing Company. Vol. 2, Number 2 (Fall 2001)). In Germany the number of catheter related blood stream infection has been estimated to ~11000 per year resulting in ~ 1000-1400 deaths and 40000 - 60000 additional patient days in the ICU (Gastmeier P, Weist K, Ruden H. Catheter-associated primary bloodstream infections: epidemiology and preventive methods.. Infection 1999;27:S1-6. The German version was published a year earlier: Infection 1998;26:S1-7.)

Devices that have the highest rate of nosocomial infection include various catheters including ports and peripheral inserted central catheters (PICC) lines used for administration of liquids to the venous system such as saline, electrolytes, medications, imaging enhancers, chemotherapy drugs, and parenteral nutritional products as well as hemodialysis and hemofiltration. Other types of catheter devices such as enteral feeding tubes, spinal catheters, various shunts, urinary catheters, peritoneal dialysis catheters, intratracheal tubes for assisted breathing as well as diagnostic monitoring arterial catheters also carry high risk of Biofilm formation.

Patients in Intensive Care Units (ICU) of a hospital are particularly at risk for nosocomial infection. Many of these patients have several invasive lines such as urinary catheters, central venous catheters as well as endotracheal tubes for assisted breathing. These acute devices are life sustaining. Patients are often in the ICU for 3 weeks or longer while being attached to various invasive life support devices. The 3 most commonly used devices in the ICU are venous catheters, urinary catheters and endotracheal tubes, which are inserted in approximately 60% of ICU patients. Various studies report that these devices cause greater morbidity, mortality, and longer length of stay in the hospital than all other causes combined. Costs attributable to device related nosocomial infections in the US range from approximately 4 to 12 billion US$ per year.

Other devices that are used chronically outside the hospital also carry increased risk of infection. A partial list of these devices includes pacemakers and pacemaker leads, heart valves, orthopedic devices such as artificial hips, implantable automatic defibrillators, artificial heart assist devices, implantable infusion pumps, drainage devices and implanted dental prosthesis.

Microorganisms or microbes (including bacteria) are the most abundant life form on earth. These microbes can be found on skin; in the air and on almost all surfaces. They are in food, water and in the mouths and other body orifices offices. However, microbes are not normally inside body tissue or cells because the healthy body has skin as a barrier to the outside world and an elaborate effective defense immune system. This immune system senses the presence of microbes, seeks them out, engulfs and kills them and finally disposes of the waste products. Living creatures such as mammals, birds, fish etc have evolved a complex system of defense to protect themselves against microbes. Microbes, which gain entrance to the body's tissue or blood supply and grow, are the cause of infectious disease and potential death. When medical devices are placed and reside in the patient, the host defense mechanism may be impaired or evaded.

William Costerton and colleagues from the Institute for Biofilm Engineering (Bozeman, MT, USA) and others have published studies, which elucidate the growth and functioning of Biofilm in many environments including the medical field. Biofilm is ubiquitous in nature and often coexist with host mammals if the host creates a blocking mechanism for entry of bacteria into the inner body space. Indwelling medical devices often provide a protected niche within the host to create a blocked off space where the microbes can maintain viability. For example, immediately after implantation of a subcutaneous device, a collagen skin begins to form and envelope the device. This skin, called a "pocket", becomes a barrier between the device and vascularized tissue. This barrier blocks the immune system's proteins and cells in the blood, from reaching the target microbes to eradicate them. The microbes can therefore proliferate without challenge. Consequently, small numbers of microbes, which were on the device at the time of implantation can grow and form quickly into Biofilm mass. Also, even if the immune system cells reach the device, the artificial surface of the device inhibits the body's immune system from attacking and eradicating attached microbes. Once the Biofilm is established, the microbes change in profound ways. Most significantly, they become much more resistant to the effects of bactericidal effects of antibiotics compared to bacteria in a planktonic form. Furthermore, many microbial life forms produce a gel matrix (called slime) as they transform into Biofilms. The slime matrix encasing the microbes is a further barrier that the antibiotics must penetrate to reach the microbes and kill them.

Biofilm can grow into a large mass. The Biofilm mass size is limited by the ability of the diffusion pathway to convey nutrients and carry away waste products from the microbes within the mass structure. When the pathway becomes overwhelmed with material, the mass stops growing. Implanted devices colonized with Biofilm microbes into the blood stream or into healthy tissue and cause infection. Because of the transformational change in the microbes within a Biofilm, the infection is very difficult to treat with the device implanted in a patient. In many clinical situations doctors decide that eradication of the infection is impossible without removal of the device. This can be a dilemma for the patient as often implanted devices are life sustaining. The risk of not effectively eradicating an infection must be weighed against the loss of function provided by the device. This is a common situation in hemodialysis treatment where catheter related blood stream infection is a high cause of death and device related infection is intractable to treat by conventional means. For these reasons, many doctors choose to remove infected catheters. Of course the patient still requires hemodialysis, which requires some type of blood access. So the patient is usually given a course of systemic antibiotics, the infected catheter is removed, a new catheter is placed in a new site and the patient is closely monitored to determine effectiveness of infection treatment. However, this is not a good long-term practice, because blood vessels often get damaged during catheter insertion and patients have only a few catheter placement sites.

Some patients do not experience an overt incident of infection even when the device is contaminated with bacteria. Patients with good immune systems can often resist bacterial assault over a considerable period of time. However, recent studies show that patient under chronic exposure to bacteria often exhibit of an increased inflammatory state as measured by C Reactive Protein (CRP) in their blood along with other markers. This condition is linked to an increase in cardiac complications and mortality. Also several other diseases are linked to chronic bacterial assault.

A further problem results from this infection. Biofilm is difficult to eradicate, requiring long-term antibiotic treatment for several weeks. Persistent long-term exposure of microbes to antibiotics imposes environmental pressure on microbes to evolve into a hardier resistant form in order to survive and reproduce. The emergence of bacterial resistance has become widespread and threatening to the health levels achieved since the use of antibiotics in the 1950s. Many countries of the world have initiated programs to reduce the use of antibiotics in animals and humans as a result.

Devices become colonized because microbes from the outside world penetrate the patient's microbial barriers. The skin is the major barrier between tissue, cells and body fluids. Openings in the body such as the mouth, nose and other body orifices incorporate various means of inhibiting the growth and passage of microbes into the inner space of the body. The surgical placement of a device into the body offer a major penetration opportunity for microbes to get inside. Even with the most rigorous aseptic controls, it is virtually impossible to prevent some numbers of bacteria from being carried into the body on outer surfaces of the devices. If the bacteria can evade the host immune system for a few hours and survive they will grow into a biofilm.

Catheters and other transcutaneous devices (i.e., devices which cross the skin barrier) carry an additional means for microbe entry. The interface between the catheters outer surface and the host tissue provides a tiny space allowing microbes to migrate into the body from outside. Additionally, the surgical procedure itself adds to the risk. Certain placement techniques tend to drag or push bacteria into the inner space of the patient. For example pushing catheters into a blood vessel using a non - cutdown surgical method may allow the catheter to rub across the patient's skin prior to entry into tissue and the blood vessel and carry in microbes from the patient's own skin. It is virtually impossible to remove all bacteria from a living patients skin.

Another example is the insertion of a urinary catheter into the urinary bladder. It must pass through the Penis (or Vagina) and into the Uretha which are known to harbor high levels of microbes. Similarly, during the insertion of Ventilation tubes, the device has considerable contact with the Mouth and Trachea, which are highly colonized with microbes.

Subcutaneous implantation of devices such as pacemakers, implantable pumps and ports, create a sanctuary for bacteria to thrive in the subcutaneous pocket encapsulating the device. The pocket isolates the device from the host tissue and blood supply to protect the host from the foreign materials and protects the microbes, which may have been deposited on the device providing a safe haven from the body's immune system.

A common but serious problem with catheters placed in the blood stream is that these devices are often used to withdrawn blood or administer blood products. Blood must flow to maintain its fluidity. In a stagnant blood pool a cascade of steps changes the blood from a fluid to a solid form (i.e., clot). So it is often the case that any blood that is left in a catheter during a time of no fluid exchange will clot. This thrombus may plug the catheter or at least fill a portion of the lumen with clot fragments causing an increase in flow resistance. It has been established that clot fragments are preferred sites for harboring microbes..

Standard catheter practice is to instill a catheter "lock" after blood flows through the catheter. The locking procedure consists of several sequential steps. Usually the lumen of the catheter is initially flushed with a small quantity of saline (i.e., normally 2 to 4 times the volume of the catheter's internal volume) to remove any blood occupying the internal space of the catheter. Then the catheter is instilled with a precise amount of liquid anticoagulant such as heparin or low concentration sodium citrate (i.e., 1 to 4 %), which is nearly equal to the internal volume of the catheter. Then the catheter is clamped shut and the sealing cap on the proximal end is put in place tightly as a redundant seal. The fluid sealed in the catheter is called a lock.

This catheter locking procedure is somewhat effective in reducing clotting compared with not using an anticoagulant in many applications. However, even with this anticoagulant lock, catheter complications of plugging and increased flow resistance are still common. The use of these anticoagulant locks does not prevent Biofilm formation within the catheter and does not prevent catheter related blood stream infection in the host.

Over the least ~15 years several attempts have been made to prevent and /or to treat infections in patients related to the use of indwelling medical devices. In central blood catheters studies have been performed and patents issued teaching the use of locking solutions including a.) Comprising antibiotics agents, b.) Antibiotics mixed with anticoagulants, c.) Antimicrobials mixed with anticoagulants d.) High concentration citrate that acts as both an anticlotting agent and a bactericidal agent and e.) Ingredients comprising Taurolidine, citric acid and sodium citrate f.) Antimicrobial and anticoagulant locks with density and viscosity additives and g.) Alcohol mixed with anticoagulants. Although these locks have some merit they do not solve the twin problem which plague most catheters which is the very high complications resulting from clotting and infection. In addition some complications are caused by the locks.

It is now clear that the use of antibiotics as a prophylaxis carries a critical harmful consequence. Most infection control experts and regulatory agencies feel that widespread chronic use of antibiotics will hasten the loss of life saving antibiotics. In 2002 in the USA, strains of Staph aureous have been discovered in two unrelated patients, which are totally resistant to Vancomycin. This has had a frightening effect on the health system practitioners, as Vancomycin had become the drug of last resort as earlier used drugs lost their effectiveness against this common pathogen. As resistant bacteria make further gains, drugs may not be able to eradicate bacteria causing fatal diseases . For these reasons, experts say that the use of antibiotics should only be used in treating life threatening diseases rather than widespread application as a preventive measure.

Although some lock compositions used have improved the art, none has eliminated both clotting and Biofilm formation inside catheters. A major factor in this result is because a full strength lock solution is usually not achieved nor retained throughout internal volume of the catheter lumen during the time interval between uses. In an unintended action, a significant volume of the lock solution leaves the catheter. This action causes blood to enter the catheter resulting in the lock's concentration to fall below the minimum level necessary to prevent clotting of that blood. Also, the lower lock concentration fails to block attachment of bacteria that have entered the catheter during use and the formation of Biofilm Experiments show that the concentration of the lock solution injected into the catheter at the time of instillation becomes greatly diluted in the distal portion of the catheter. This result is primarily caused by 3 different factors as defined below:

During instillation of the lock solution into a catheter; typically with a syringe; the lock solution flows according to well-understood principles in fluid engineering. Normal fluids flow in either a "laminar flow" or "turbulent flow" regime. In a manual syringe instilled lock using typical lock liquids, the flow is typically in the laminar flow regime. In laminar flow, the flow streams (or particles) in the tube, flow in parallel lines in the direction of the center axis (i.e., flow lines do not mix) and the flow velocity is maximum at the center of the tube and near zero (i.e., stagnant flow) at the wall of the tube. At points between the center and the wall, the flow velocity is between zero and maximum velocity following a well-known engineering formula, which is a parabolic curve (Nichols WW, O'Rourke MF. The nature of flow of a fluid. Nichols WW, O'Rourke MF, editors. McDonald's Blood Flow in Arteries. Edward Arnold, A division of Hodder&Stoughton, 1990:12-53). This means that locking solution will be leaving the center portion of the catheter lumen well before fluid near the walls has reached the end of the tube. The inventor has measured this effect in a typical hemodialysis catheter and saline and found that lock fluid will be exiting the distal tip even when only about half of the fluid needed to fill the lumen has been injected into the catheter (Polaschegg HD, Shaw Ch. Catheter locking solutions: safety and efficacy of composition and methods. ASAIO J 2002;48:178). The consequence is that a significant amount of lock fluid (~15%) is spilled into the patient when the amount instilled is equal to the volume of the catheter lumen, which is the conventional technique of instilling locks. Also, resulting average tip concentration across the full area of the lumen is considerably lower than the instilled concentration. To achieve a concentration of lock solution equal to the concentration instilled, significantly more lock solution volume (~20%) needs to be instilled than the volume of the actual catheter lumen. This would of course result in considerable spillage into the patient. Further more, under various amounts of fluid instilled, the concentration of the lock solution at the inside wall surface has a considerable lower concentration than the concentration at the center of the tube although it is more important to achieve the effects of anticoagulation and biocidal effects at the lumen wall than in the center. The purpose of the lock is to prevent adherence of clots or microbes to the surface of the lumen.

Density differences between a catheter lock and the host blood will cause flow to take place between the catheter and the bloodstream. For example, in a vertical catheter filled with lock solution and immersed in blood and with the proximal end sealed tightly and the distal tip open and with a lumen lock density slightly higher than the blood, locking fluid will exit the lumen and blood from the blood stream will enter the lumen to make up for fluid leaving the catheter (Polaschegg HD, Sodemann K, Estabrook B. Investigation of the effect of catheter side holes on flow properties and outwash of the locking solution. Proceedings of the 2nd Symposium on Angioaccess for Hemodialysis. Vascular Access Society, Maastricht, 1999: 195). In vivo, with the patient changing orientation during the course of the day (i.e., standing and sleeping, etc) new conditions arise and flow distribution is reset to the new conditions. The shifting of fluid in and out ceases when the catheter reaches mixture of blood and lock in density equilibrium.

Tip geometry is different among the various commercially available catheters. Many catheters, especially dialysis catheters incorporate radial directed side holes near the distal end and other catheters do not have side holes. It has been observed that side holes speedup the mixing of blood and lock solution occurring at the distal end and it is thought that side holes may help anchor thrombus to the catheter by a mechanical interlocking making it more difficult to displace and achieve good flow rates. Nevertheless, in all catheters considerable tip mixing occurs due to dynamic fluid flow in blood vessels. Central blood catheters have their tips near the beating heart, which influences fluid dynamic forces and catheter motion. The forces driving blood/lock exchange is especially severe when the catheter tip resides in the Right Atrium of the Heart. The Right Atrium is the collection chamber to the heart. It is in constant motion accepting flow from 2 opposite directions (i.e., blood from the lower part of the body and from the upper part). Each pumping cycle of the heart (i.e., approximately 1 beat per second at rest) causes abrupt movement of the atrium as the Right Atrium fills and collapses. Blood rushes from the atrium to fill the right ventricle for each beat producing a pressure pulse of approximately - 5 to + 5 mm of water in the right atrium during the cardiac cycle. Movement oscillations and the pressure pulses cause further perturbation of the fluid conditions in and around the catheter producing flow in and out of the tip passages in the catheter.

This dilution reduces the active agents to that provide antimicrobial and anticoagulation action to the lock. There is no doubt that this dilution phenomena is occurring. In many dialysis clinics in Europe and North America, a heparin concentration of 5,000 units per mL is placed in the catheter. This is approximately 1000 times higher level than required to prevent coagulation in blood and considerably more than required for anticoagulation of the patient. Yet even at this high level, most catheters still show major clot development at the tip, which can be seen when the lock solution is removed from the catheter during the preparation for a new hemodialysis treatment (i.e., typically occurs every 2-3 days). To find out if the powerful anticoagulant heparin was spilling from the catheters, clotting time testing was performed on typical hemodialysis patients immediately after the lock instillation. Doctors were surprised at the finding that lock spillage does occur in significant amounts causing patient systemic clotting times to become elevated. It appears that most hemodialysis patients are inadvertently anticoagulated for several hours after a lock instillation when locked with heparin (Karaaslan H, Peyronnet P, Benevent D, Lagarde C, Rince M, Leroux-Robert C. Risk of heparin lock-related bleeding when using indwelling venous catheter in haemodialysis.. Nephrol Dial Transplant 2001;16:2072-4 and Moritz, M., Vats, A., and Ellis, D. Systemic Anticoagulation and Bleeding in Children with Hemodialysis Catheters. Pediatr. Nephrol (2003) 18:68-70).

Systemic injection of the lock solution had tragic consequences for at least one patient. Hemodialysis catheters are intentionally placed so that their tips are directly in the right atrium of the hearts. This patient was instilled with high concentration Citrate Lock Solution (i.e., ~ 35%) leading to heart arrest. The lock solution is described in PCT Patent Application WO 00/10385 published 3 March 2000. The product was commercialized in the USA without US Food and Drug Administration (FDA) approval and a death occurred soon afterwards. FDA issued a safety alert warning of the use of high concentration citrate as a lock in central bloodlines and the manufacturer recalled the product, which has not returned to market. FDA limited the concentration of citrate for any venous infusion procedures to 4%. Citrate is a powerful chelator of Calcium ions. The presence of Calcium ions is necessary for muscle contraction and is involved in the pacemaker electrical signal cascade in heart muscle contraction synchronization. It is thought that Citrate spilled out of the catheter into heart muscle. The citrate chelated calcium ions in the heart causing heart dysfunction and death.

This accident illustrates the importance of a medical safety principle incorporated in the European Medical Device Directive. The Directive requires that risk analysis be performed to demonstrate that the patients life is not at risk if a foreseeable fault or user error occurs. For example, a single fault would be a nurse inadvertently giving a patient a second dose of lock solution.

An antimicrobial lock (PCT Patent Application WO 00/01391) comprising Taurolidine as an antimicrobial agent, citrate as an anticoagulant and citric acid as a stabilizing agent was evaluated in Hemodialysis patients with Ports and Catheters in a European Clinical Trial. Excellent results were reported for reducing catheter related infection nearly to zero (Sodemann, K., Polaschegg, H., Feldmer, B., Two Years Experience with Dialock and CLS (A New Antimicrobial Lock Solution), *Blood Purif.,* 2001; 19:251-254). In a subsequent controlled study using the locking solution, which measured both infection and thrombosis, the results again showed substantial reduction in catheter related blood stream infection compared to conventional heparin lock. However, they also observed 3 times more patients had flow complications with the taurolidine lock than with a conventional heparin lock (Allon M. Catheter Lock Solution (CLS) for Prophylaxis of Dialysis Catheter-Related Bacteremia (CRB): A Pilot Study. J Am Soc Nephrol 2002;13:34A)

Taurolidine is a molecule having several properties, which make it uniquely suited to be an excellent antimicrobial agent acting in or around the local environment of in vivo medical devices (Polaschegg HD. Taurolidine, a new antimicrobial catheter lock solution. Dialysis Times 2000;7:1,8 ). These properties are:
A long history of safe use in several European countries. It has a high margin of safety if ingested or injected. It is approved for use as a peritoneal lavage for treatment of peritonitis and as a drug for treatment of osteomyletis
Broad spectrum effect on killing bacteria and is also effective in killing certain fungi associated with medical device infections
No reported evidence has ever been published showing the emergence of bacterial resistance Large overdoses into the systemic circulation do not produce a pharmacological response Relatively small molecular size for high diffusion rates through the slime gel encasing the Biofilm microbes
Inactivates endotoxins released by dead bacteria(i.e., present in dead bacteria)

Taurolidine has other properties that detract from its capability as a local antimicrobial agent. These are:
The minimum inhibitory concentration (MIC) value of Taurolidine necessary to kill bacteria and fungi is much higher than that of antibiotics used in medicine. The saturation concentration of Taurolidine in water (i.e., without precipitation occurring) is not very much higher than the MICs.
Taurolidine needs to be in contact with microbes several minutes to several hours to achieve high kill ratios compared to disinfectants which kills microbes in seconds to a few minutes Water is the preferred solvent for Taurolidine and /or Taurultam

Catheters are commercially available that have coatings applied to protect against antimicrobial infection. Most of the coatings contain antibiotics, which is not a good choice because it contributes to the steady emergence of bacterial resistance. Also, the coatings offer antimicrobial protection only for a for very short time span as they are used up typically in 10 5-20 days. This is insufficient for chronic catheters as, e.g., used for dialysis, TPN, and urinary catheters for nursing home patients.

There remains a need for methods and compositions for prevention of thrombus and Biofilm formation in fluid delivery systems and other indwelling medical devices. The need grows more critical as more invasive devices and medical procedures are used while the emergence of bacteria resistant to antibiotics accelerates.

Various formulations of gels containing antimicrobial substances have been described in the patent literature. None of these patents, however mentions thixotropy or the use of gels as catheter lock solution or a gel that can be injected into the blood stream.

EP 1103251 (Goldschmidt AG) describes a gel formulation containing Taurolidine as antimicrobial substance for the treatment of acne.

WO 9403174 (Geistlich) describes formulations containing Taurolidine as antimicrobial substance for the treatment of dentoalveolar infections. The formulations include emulsions, gels, rinse solutions with or without thickening agents. The purpose of using such agents is delayed release of the antimicrobial substance.

US 4,905,700 (Wokalek) describes the composition and application of hydro gel sheets for coupling of ultrasound transducers to the skin. This sheet may contain an antimicrobial substance (Taurolidine) for maintaining sterility.

US 4,587,284 (Lueissi) describes the preparation of hydro gels able to take up water or aqueous solutions. These solutions may contain Taurolidine for maintaining sterility of the final product. The hydro gels are formed into blocks or the like.

US 6,451,003 (ProsI) describes methods for overcoming infections in the pocket surrounding implantable devices. Among the materials and methods described are gels, soluble in body fluids. The gel is used for slow release of an antimicrobial substance. This patent also describes the use of catheters but only as part of the implanted device, the application of the gel is limited to the exterior of the implanted device.

Other patents describe formulations containing Taurolidine for use as liquid catheter locks.

EP 1040841 (Pfirrmann) describes a liquid formulation containing Taurolidine that is used as catheter lock solution ("sealing solution") between treatments with the goal of preventing infections and thrombosis caused by adhesion of blood components to the catheter lumen surface.

US 6,166,007 (Sodemann) describes formulations containing Taurolidine which are used as catheter lock solutions. The discussion of previous art mentions the use of gels but only in conjunction with applications not related to direct contact with the blood circulation.

WO 9828027 (Lehner) describes a catheter lock solution containing Taurolidine.

US 20020010438 (Finch) describes a catheter locking solution containing a mixture of a lower alcohol with other anti-microbial substances.

### Summary of the invention:

The inventor has found that several new elements may be used to particular advantage in combination with Taurolidine and/or Taurultam to prevent clotting and Biofilm formation or the elements can be combined with other antimicrobial agents. In accordance with the present invention a composition is used to prevent the attachment and subsequent growth of Biofilm on surfaces along with the prevention of clotting in passages of indwelling medical devices. These elements separately and particularly in combination make a more functionally ideal catheter locking material, which solves the unmet need for a better catheter lock solution.

According to claims 1 and 8 to 10, in the present invention a gel with thixotropic properties is used to keep the lock inside the catheter and not spill out during the time interval between uses. This is accomplished by making a hydrogel matrix as a drug delivery vehicle containing a biocompatible antimicrobial agent alone or with another active agents, which may be useful for particular purposes. The hydrogel matrix is biocompatible and, biodegradable in the bloodstream. The matrix can be a hydrogel (e.g., pectin, gelatin, etc), a protein (e.g., collagen, hemoglobin, etc), a colloidal substance (e.g., serum albumin etc), an emulsion or other adjuvant. Preferably, the matrix shall have structural integrity and be thixotropic while instilled in a catheter during its locking function, which may be for several days to a few weeks. Thixotropy is a property, which is exhibited by certain gels. It is a property characterized by a solid or semisolid substance that when shaken, stirred or subject to high shear forces becomes fluid like and can flow and then returns to the semisolid state when the forces and/movement are stopped. Alternatively, the gel could have the properties similar to that of the colloidal dispersion similar to a sauce called "Ketchup" (i.e., tomato sauce), which resists movement, or flow until a high shear force is imparted to the fluid and then it flows easily. It has been observed that certain thixotropic hydrogel substances can be easily injected into a catheter and the gel does not have the variable velocity exhibited with the parabolic velocity profile of a laminar flowing liquid. Instead the semisolid gel moves into the lumen and travels through it as a cohesive rod-shaped mass of material. The result is that one can completely fill the catheter with this gel and obtain a uniform concentration without dilution near the tip without the overfilling and corresponding dumping of lock into the patient.

This gel is retained in the catheter even when the catheter tip is flapping (i.e., similar to a flag moving rapidly in the wind) in the right atrium or subject to density differences or being subjected to moderate pressure variation pulses. However, the gel can easily be withdrawn from the catheter with a syringe. It has been observed that once a threshold level force is achieved the lock substance flows freely. The tendency to spill out and to mix with blood was greatly reduced during experiments. This substance achieves nearly a 100% lock concentration at the tip and will prevent the mechanical intermixing of blood in the catheter. In practice, the retained lock does not allow blood to enter the catheter. Accordingly, with the absence of blood, no clotting or thrombus formation will occur within the catheter. Other ingredients may be added to the gel matrix to provide further functional benefit. The preferred antimicrobial is Taurolidine, which can be added to the matrix as a micro particle powder, or encapsulated in liposomes, microspheres, or nanospheres. It should be appreciated that use of a thixotropic gel lock material, many different active agents and drugs including sterileants, lysing agents (such as Urokinase), imaging enhancers, catheter surface modifiers, antibiotics and antimicrobial chemicals can be formulated or mixed into the lock to provide additional functional characteristics and even drugs which can be delivered from the catheter lock a through diffusion mode through the catheter side wall. Mixing gel with a powder results in a saturated solution of the substance contained in the powder in the water phase of the gel with the remaining not-dissolved substance acting as a reservoir. As the dissolved substance leaves the gel by diffusion, additional substance is dissolved from the non-dissolved reservoir space. This is especially useful for long term application when the gel is filled into body cavities, e.g., pockets of implantable devices.

Hydrogel is a three-dimensional network with a large amount of water giving them good biocompatibility with material consistency that is soft solid - like with high diffusive properties to gases, chemicals and proteins . Most research on hydrogels from the beginning (Wichlere O., Lin D. Nature 1960; 185:117-8) has been geared towards biomedical applications.

A wide variety of biomedical applications has been developed since then including soft contact lens, corneal implants, ophthalmic antibiotics for dry eyes, drug delivery depots, artificial breast prosthesis, tissue engineering scaffolds, urinary catheter coatings, vaginal gels, wound-healing devices, injectable antibiotic forms for treating periodontal disease and skin moisturizers to name only a few.

Materials comprising hydrogels include natural polymers including serum albumin, collagen, or alginates as well as synthetic polymers such as polyvinyl alcohol, poly (ethylene oxide) or poly (hydroxyethylene) and polyelectrolytes, such as poly(acrylic acid), poly(styrene sulfonate), and carboxymethylcellulose (CMC).. Serum albumin has antimicrobial properties, which would enhance an antimicrobial lock mixture. It is a material found in blood and is a useful viscosity adjuster

The desired makeup of the hydrogel catheter lock is as a support material providing bulk cohesive integrity necessary (i.e., thixotropic characteristics) to retain the lock within the catheter between catheter uses. It is important that if a fault condition were to occur, wherein the gel is accidentally injected into the blood stream, the bulk gel will dissolve and not block flow in the small vessels of the blood stream. Hydrogel biodegradation is also important in other misapplications where the gel materials are only intended for short periods such as the controlled release of drug from a depot. For example, it is known that Hydrogels comprising boronic acid polymers and poly(vinyl alcohol) degrade in the presence of glucose (Lee S, Park K. Glucose-Sensitive Phase-Reverseable Hydrogels. Ottenbrite R, Huang S, Park K, editors. Hydrogels & Biodegradable Polymers for Bioapplication. American Chemical Society,1996:chapter 2)
Upon further addition of water to a hydrogel formed from a hydrophilic polymer, polymer-polymer contacts are broken and individual polymer molecules are dissolved in water.

The first embodiment of this invention is to teach methods and formulation of a novel family of catheter lock materials that encompass a wide variety of active properties that fit the particular clinical application providing lock be retention within the catheter during the time the catheter is not being used. This embodiment is to provide a gel lock biocompatible, safe and thixotropic and comprising the active agent(s) which prevents clotting and Biofilm formation within the catheter.

Another embodiment of the invention is a novel antimicrobial formulation, which combines Taurolidine with Salicylic acid or Sodium Salicylate in an aqueous solvent. Salicylic Acid and Sodium Salicylate are drugs that have been used with antibiotic locks in catheters to enhance the biocidal action of the antibiotic alone and to inhibit the attachment of microbes to surfaces. This last attribute is especially important because the initiation of a Biofilm expression and growth require that the individual bacteria must first attach themselves to the underlying surface. By stopping attachment, Biofilm formation is blocked.

Salicylic acid and Sodium Salicylate are classified as analgesics and Non Steroid AntiInflammatory Drug (NSAID). They are used to treat pain and fever, swelling, and joint pain of arthritis or rheumatism and are available as prescription and non-prescription drugs and are combined with other agents to make up drugs.

Sodium salicylate has been demonstrated to have remarkable antibacterial activity, including the ability to enhance the activities of certain antibiotics. This drug inhibits adherence, growth and Biofilm formation. A study indicates that the efficacy of Vancomycin against Staphylococcus epidermidis is significantly enhanced when salicylate is added. Staphylococcus epidermidis is a common etiologic agent of catheter related infection. (Polonio RE, Mermel LA, Paquette GE, Sperry JF. Eradication of biofilm-forming Staphylococcus epidermidis (RP62A) by a combination of sodium salicylate and vancomycin. Antimicrob Agents Chemother 2001;45:3262-6)

The inclusion of 5 milli Molar salicylic acid (SAL) in a medium inhibited both growth and Biofilm production of Staphylococcus epidermidis by up to 55%. This effect was determined to be a reduction in the production of Biofilm rather than a reduction in the bacteria before formation of Biofilm. (Muller E, Al-Attar J, Wolff AG, Farber BF. Mechanism of salicylate-mediated inhibition of biofilm in Staphylococcus epidermidis. J Infect Dis 1998;177:501-3)
Slime-inhibiting compounds including salicylic acid and other NSAID inhibit the growth of microorganisms on catheters and other medical devices. Coating silicone rubber catheters with salicylic acid makes them resistant to bacterial attachment which supports the concept of preventing Biofilm formation and preventing catheter related infection (Farber BF, Wolff AG. Salicylic acid prevents the adherence of bacteria and yeast to silastic catheters. J Biomed Mater Res 1993;27:599-602).

A further embodiment is that achievement of prophylaxis of device related infection must encompass the entire life cycle of the device in proper manner to be effective. Although this may seem obvious, it is not being practiced in hospitals and clinics around the world. This embodiment of the invention envisions that effective methods must protect against contamination and loss of sterility of the device during storage, while removing the device from its package, during placement of the device into the patient and while operating and maintaining the device in vivo. For example, Ventilator tubes for assisted breathing have a very high infection rate in the ICUs of Europe and the USA. They account for the highest mortality in the ICU due to nosocomial infection. The numbers of deaths and the cost of treating these infections are quite substantial as - 60% of patients admitted to the ICU receive Ventilator breathing tubes. The infection that commonly occurs is called Ventilator Associated Pneumonia (VAP).

Endotracheal tubes are often installed in the ICU to patients who are sedated under conditions that are often less than perfect. During installation, the tubular device is inserted in the mouth and pushed down into the trachea to the respiratory tract. The insertion causes the tubular device to rub across surfaces of the mouth & oropharynx, which are heavily colonized with bacteria. Once the ventilator tube is in place, the cuff is inflated to make a seal around the outside surface of the tube and the trachea. It is known that aspiration around this seal occur continuously as breathing cycles of the ventilator milk secretions around the cuff of the tube. Patient's stay in the ICU for an average of 8 days on a ventilator. During this time the patients remain sedated and cannot cough which otherwise helps to expel contaminates and bacteria from the throat and lungs. Bacteria in the mouth and throat can move down the outside of the ventilator tube or the inside surface of the throat and finally reach the alveoles causing pneumonia which is very difficult to treat successfully.

This embodiment of the invention teaches methods, which reduce the level of viable bacteria into the lungs during the installation of the device and during use of the device. Prior to inserting the Ventilator tube into the patient, the tube shall be copiously coated with a slippery antimicrobial hydrogel. Some of the antimicrobial gel coating will be transferred to the trachea as the tube is being inserted into place. Also, during the insertion procedure, a light spray of antimicrobial solution is directed ahead of the tubes distal end to help coat the trachea surfaces with antimicrobial solution or gel to lessen the amount of viable microbes being pushed into the airway passages. A further embodiment is to use Taurolidine as an antimicrobial to further deactivate resulting endotoxins, liberated from the dead or impaired bacteria.

A further improvement is the incorporation of an active antimicrobial delivery system built into the ventilator tube. This system is activated after insertion to deliver an antimicrobial solution, which bathes the ventilator tube's outer surfaces and the inner surface of the throat with a biocompatible antimicrobial agent. This stops viable bacteria from migrating along the tube into the lungs and causing pneumonia. A nurse can refill the delivery system reservoir periodically. The schedule of delivery of this bathing action is controllable and can be performed automatically. The type of mechanical or electrical apparatus, which is outside the body, is well known by medical device engineers.

Modification of the NCCLS Test Method "Methods for Dilution Antimicrobial Susceptibility Tests for Bacteria That grow Aerobically": Approved Standard- Fifth Edition, NCCLS Document M7-A5 according to a pre-approved protocol. The test organisms used in the experiments were Staphylococcus aureus ATCC # 700788, Staphylococcus epidermedis ATCC # 27626, Enterococcus faecalis ATCC # 700802, Pseudomonas aeruginosa ATCC # 27313, and Candida albicans ATCC # 90029. The test batch solutions were tested in duplicate in undiluted and diluted state. Each sample was inoculated with the microorganism to achieve a final concentration of approximately 5x10⁸CFU/ml (i.e., CFU is colony forming units). Measurement of organism concentration to determine kill was after 24 hours exposure and the results are presented as a mean log reduction in viable bacteria.

The make up of the 3 batches were as shown in the following table

**Table 1: Test Lock Solution Formulations**

| | Batch 1 | Batch 2 | Batch 3 |
|---|---|---|---|
| Taurolidine (Wt./Wt.) | 1.5% | 2% | 2% |
| Sodium Salicylate | 0.1 Molar | 0.1 Molar | 0.4 Molar |
| pH | 6,3 | 6,1 | 6,1 |
| PVP (Wt./Wt.) | 5% | 5% | 5% |
| Balance | Water | Water | Water |

The killing death rate results as expressed as a mean log reduction isare shown in the tables 2 - 4 for the 3 batches of the lock formulation.

**Table 2: Mean Log Reduction in CFU**

| **Batch I** (1.5% Taurolidine & 0.1 M Sodium Salicylate) | | | | | |
|---|---|---|---|---|---|
| | **Staph aureus** | **Staph epidermedis** | **Enterococcus faecalis** | **Candida albicans** | **Pseudomonas aeruginosa** |
| **No Dilution (100%)** | >5.0 | >5.0 | 3.2 | 3.3 | >5.0 |
| **1:2 Dilution (50%)** | >5.0 | >5.0 | 1.4 | 0.3 | >5.0 |
| **1:10 Dilution (10%)** | 3.0 | 4.4 | 1.9 | No Reduction | >5.0 |
| **1:20 Dilution (5%)** | 1.6 | 1.7 | 0.8 | No Reduction | No Reduction |

**Table 3: Mean Log reduction in CFU**

| Batch II (2 % Taurolidine & 0.1 M Sodium Salicylate) | |
|---|---|
| | **Candida albicans** |
| **No Dilution** | >4.7 |
| **(100%)** | |
| **1:2 Dilution** | >2.6 |
| **(50%)** | |
| **1:10 Dilution** | No Reduction |
| **(10%)** | |
| **1:20 Dilution** | No Reduction |
| **(5%)** | |

**Table 4: Mean Log reduction in CFU**

| **Batch III** (2 % Taurolidine & 0.4 M Sodium Salicylate) | |
|---|---|
| | **Candida albicans** |
| **No Dilution** | >4.7 |
| **(100%)** | |
| **1:2 Dilution** | **>4.7** |
| **(50%)** | |
| **1:10 Dilution** | No Reduction |
| **(10%)** | |
| **1:20 Dilution** | No Reduction |
| **(5%)** | |

### Catheter lock spillage experiments

In a first test series it was demonstrated that powder mixed with a hydrogel remaines uniformly distributed in the gel.

In a second series of test it was shown that the gel moves plug like in the catheter lumen when injected and that it remains there after the end of injection even when the distal side is vented while the proximal side (tip) is in a water bath. By measuring pressure relaxation it was possible to demonstrate the thixotropic property of the gel.

The part of the curve between 0 and 0.5 [min] in the horizontal time scale shows the negative pressure followed by an equilibration phase during which the pressure is zero. The system is than compressed but the pressure equilibrates quickly when the glycerin solution is forced out of the syringe body (22) via the catheter (26) into the vessel (30). The process stops when the pressure in the syringe (22) is again zero.

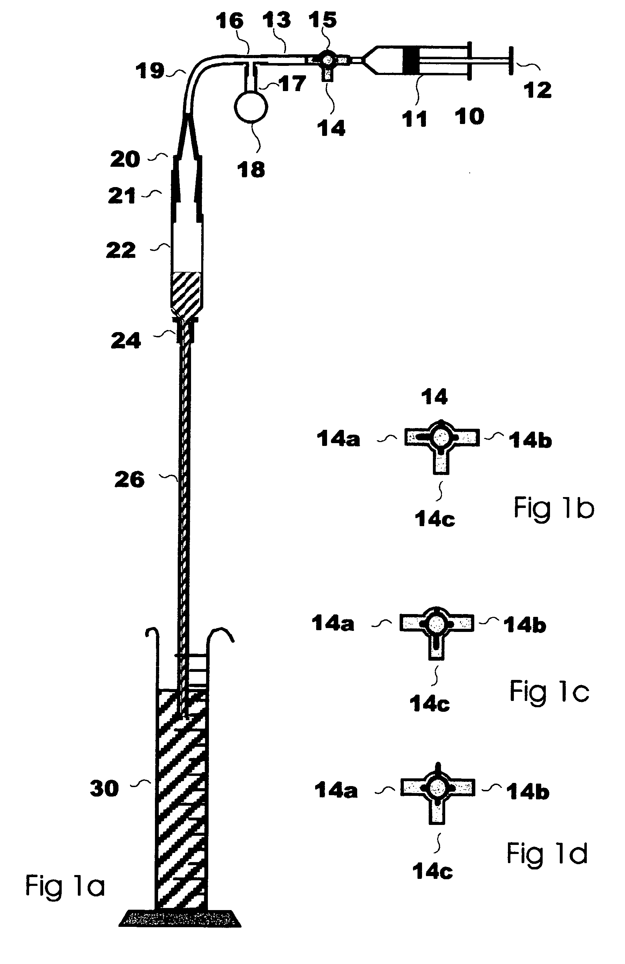

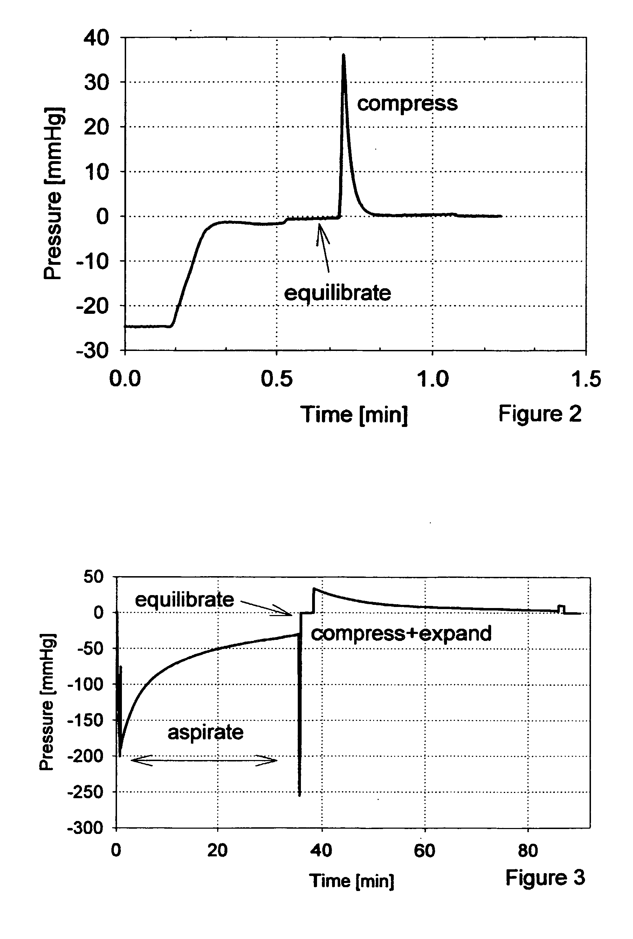

Figure 3 shows the measurement results for gel sample no2 1 part GlattLook +2 parts water. Initially the syringe (22) was put under negative pressure by retracting plunger (12) in syringe (10) which was then fixated in the final position. The pressure was then allowed to equilibrate slowly during the next 35 minutes while the test sample was aspirated. In order to bring the level in the syringe (22) to 3mL the negative pressure was briefly increased which is visible as negative pressure peak in the tracing at approx. 35 min. After that the system was briefly equilibrated and the tip of the catheter was put into vessel (30). The system was compressed to approx. 40 mmHg, the cock was put into "pressure holding" position and the system was allowed to equilibrate while gel was flowing out of the syringe into the vessel (30).

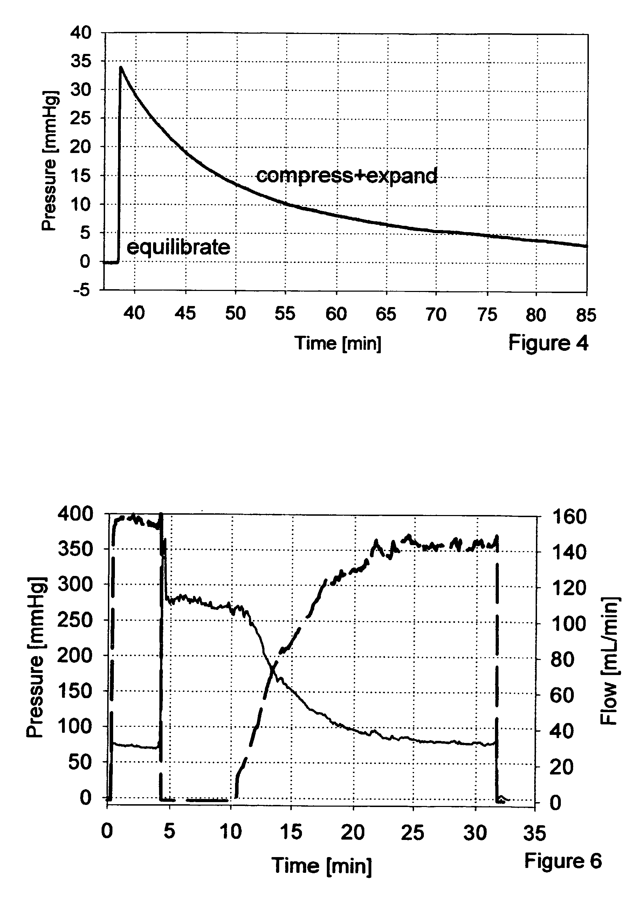

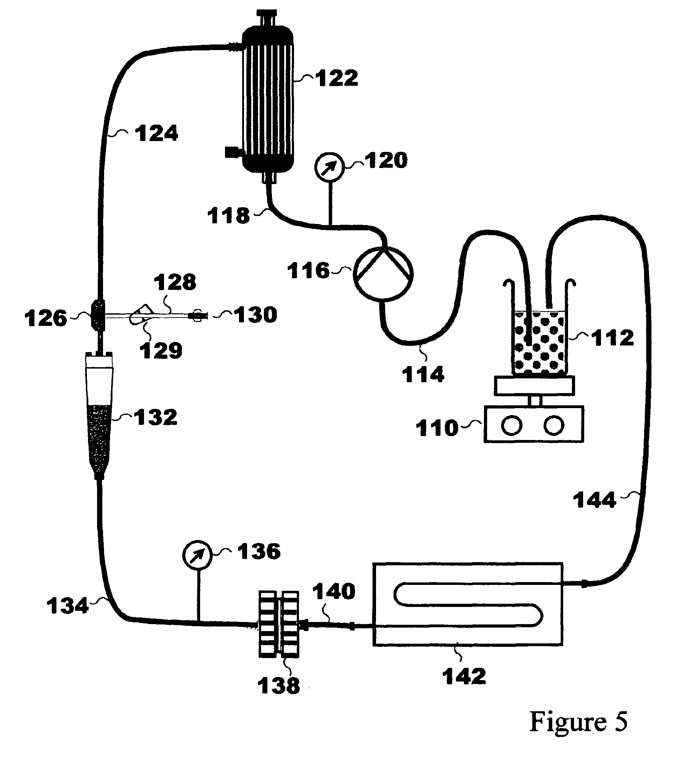

Figure 4 shows the second half of figure 3. At 85 minutes the pressure in the system was 3.5 mmHg and no outflow could be observed from the catheter. Because the system was neither non-compliant nor absolutely air tight, the observed final pressure is a lower limit for the minimum pressure for flow.

For the third series of tests the hydraulic setup described by figure 5 was used.
Water in a baker (112) is heated to 37°C by a heater-stirrer (110). The gear pump (116) pumps water from the baker (112) through line (114) and line (118) to the pre-filter (122). The pressure before the pre-filter is measured by the pressure transducer (120) which is connected to a computer (not shown). A high-flux dialyzer FX50 (Fresenius Medical Care Deeutschland) was used as pre-filter (122). The pre-filtered water leaves the filter (122) through line (124). The two other connectors of filter (122) are capped. Filtered water flows through the injection port (126) to drip chamber (132) and line (134) to the test filter (138). From the test filter (138) water flows through line (140) to the Coriolis flow meter (142) and back through line (144) to the beaker.
Injection line (128) branches from injection port (126). The injection line (128) can be clamped with the line clamp (129). Injection line (128) terminates with a female luer-lock (130).
From line (134) branches a line to "filter pressure" sensor (136).
Test filter (138) consists of a 25 mm filter holder (Sartorius AG, Germany: Filtratisonsvorsatz 25mm order no 16517) with a 5µm mixed cellulose ester membrane filter (Advantec MFS, Inc. CA, USA cat.no: A500A025A). The flow meter (142) is a Coriolis flow meter from Danfoss (Denmark) type MASS2100 DI 3 with a low flow cutoff of ~ 12 mL/min.

For the tests flow in the circuit was established with the help of a gear pump (116) which was operated at constant driving voltage resulting in a flow of approximately 150 mL/min. Initially the clamp (129) remained closed. The pressures and flow were continuously recorded by the computer. A 0.2 mL sample was aspirated into a 1 mL syringe. The syringe was connected to port (130) the clamp (129) was opened, the sample injected and the clamp (129) closed again. A 20 mL syringe filled with water was connected to port (130), the clamp (129) opened, the water injected and the clamp (129) closed again.

The result of the test measurement with the Glatt-Look gel diluted 1+2 shows figure 6. Figure 6 shows the pressure measured by the "filter pressure" sensor (136) (left axis, full line) and the flow measured by the Coriolis flow meter (142) (right axis, dashed line) versus time. Flow is established for the first 4 minutes at - 155 mL/min and the filter pressure id - 75 mmHg. At 4.5 minutes 0.2 mL gel followed by 20 mL of water were injected as described above. Flow and pressure show a short peak and flow drops below the threshold of -12 mL/min and is indicated as zero in the tracing. Flow, however, could be observed at the outflow of line (112). Pressure increases after injection to ~275 mmHg. After - 7 minutes at time 11 min flow begins to increase and filter pressure begins to drop. Both approach the original value at time 25 min. Because the water used was not free of small particles the base line was not achieved. This has been found out during tests performed with water only.

### Interpretation of test 3:

In clinical practice it may happen that gel gets injected. It will be blocked by the lung. As the test shows the gel will dissolve within minutes into particles smaller than 5 µm which will not block capillaries in the circulation before they are finally dissolved and metabolized. The injected amount was only 10% of the amount that may be injected erroneously. The surface area of the lung capillary bed, however, is more than a factor 1000 larger than the surface area of the test filter.

## Claims

1. Use of a thixotropic gel as a catheter locking composition.

2. Use according to claim 1, wherein the thixotropic gel is biocompatible and biodegradable in the bloodstream.

3. Use according to claim 1 or 2, wherein the thixotropic gel comprises one or more compounds/compositions selected from hydrogel, microgel, protein, colloidal substance, emulsion, synthetic polymers, and polyelectrolytes.

4. Use according to one of claims 1 to 3, wherein the thixotropic gel comprises one or more compounds/compositions selected from pectin, gelatin, collagen, haemoglobin, serum albumin, alginates, polyvinyl alcohol, poly(ethylene oxide), poly(hydroxyethylene), poly(acrylic acid), poly(styrene sulfonate), and carboxymethylcellulose.

5. Use according to one of claims 1 to 4, wherein the thixotropic gel comprises an antimicrobial agent.

6. Use according to claim 5, wherein the antimicrobial agent is taurolidine, taurultam or a mixture thereof.

7. Use according to claim 5 or 6, wherein the thixotropic gel comprises salicylic acid or one of its salts and/or a medically acceptable anticoagulant agent.

8. Method of locking a catheter, including the step instilling a catheter locking composition into a catheter, the catheter locking composition being a thixotropic gel.

9. Method of unlocking a catheter locked by a method according to claim 8, including the step of withdrawing the catheter locking composition from the catheter with a syringe.

10. Kit of a medicinal catheter device, comprising
a catheter and
a catheter locking composition being a thixotropic gel.

## Patentansprüche

1. Verwendung eines thixotropischen Gels als eine Katheterverschließzusammensetzung.

2. Verwendung nach Anspruch 1, bei der das thixotropische Gel biokompatibel und bioabbaubar im Blutstrom ist.

3. Verwendung nach Anspruch 1 oder 2, bei der das thixotropische Gel umfasst eine oder mehrere Verbindungen/Zusammensetzungen, ausgewählt aus Hydrogel, Mikrogel, Protein, kolloidale Substanz, Emulsion, synthetische Polymere und Polyelektrolyte.

4. Verwendung nach einem der Ansprüche 1 bis 3, bei der das thixotropische Gel umfasst eine oder mehrere Verbindungen/Zusammensetzungen, ausgewählt aus Pektin, Gelatine, Collagen, Hämoglobin, Serumalbumin, Alginate, Polyvinylalkohol, Poly(ethylenoxid), Poly(hydroxyethylen), Poly(acrylsäure), Poly(styrolsulfonat) und Carboxymethylzellulose.

5. Verwendung nach einem der Ansprüche 1 bis 4, bei der das thixotropische Gel ein antimikrobielles Mittel umfasst.

6. Verwendung nach Anspruch 5, bei der das antimikrobielle Mittel Taurolidin, Taurultam oder eine Mischung davon ist.

7. Verwendung nach Anspruch 5 oder 6, bei der das thixotropische Gel umfasst Salicylsäure oder eines ihrer Salze und/oder ein medizinisch annehmbares Antikoagulanzmittel.

8. Verfahren zum Verschließen eines Katheters, umfassend den Schritt des Instillierens einer Katheterverschließzusammensetzung in einen Katheter, wobei die Katheterverschließzusammensetzung ein thixotropisches Gel ist.

9. Verfahren zum Öffnen eines Katheters, der durch ein Verfahren nach Anspruch 8 verschlossen ist, umfassend den Schritt des Herausziehens der Katheterverschließzusammensetzung aus dem Katheter mit einer Spritze.

10. Kit einer medizinischen Kathetervorrichtung, umfassend
einen Katheter und
eine Katheterverschließzusammensetzung, die ein thixotropisches Gel ist.

## Revendications

1. Utilisation d'un gel thixotrope en tant que composition de verrouillage de cathéter.

2. Utilisation selon la revendication 1, dans laquelle le gel thixotrope est biocompatible et biodégradable dans la circulation sanguine.

3. Utilisation selon la revendication 1 ou 2, dans laquelle le gel thixotrope comprend un(e) ou plusieurs composés/compositions sélectionné(e)s parmi de l'hydrogel, du microgel, une protéine, une substance colloïdale, une émulsion, des polymères synthétiques et des polyélectrolytes.

4. Utilisation selon l'une des revendications 1 à 3, dans laquelle le gel thixotrope comprend un(e) ou plusieurs composés/compositions sélectionné(e)s parmi de la pectine, de la gélatine, du collagène, de l'hémoglobine, de la sérum-albumine, des alginates, de l'alcool polyvinylique, du poly(oxyde d'éthylène), du poly(hydroxyéthylène), du poly(acide acrylique), du poly(sulfonate de styrène), et de la carboxyméthylcellulose.

5. Utilisation selon les revendications 1 à 4, dans laquelle le gel thixotrope comprend un agent antimicrobien.

6. Utilisation selon la revendication 5, dans laquelle l'agent antimicrobien est de la taurolidine, du taurultam ou un mélange de ceux-ci.

7. Utilisation selon la revendication 5 ou la revendication 6, dans laquelle le gel thixotrope comprend de l'acide salicylique ou un de ces sels et/ou un agent anticoagulant médicalement acceptable.

8. Procédé de verrouillage d'un cathéter, comprenant l'étape consistant à instiller une composition de verrouillage de cathéter dans un cathéter, la composition de verrouillage de cathéter étant un gel thixotrope.

9. Procédé de déverrouillage d'un cathéter verrouillé par un procédé selon la revendication 8, comprenant l'étape consistant à retirer la composition de verrouillage de cathéter du cathéter avec une seringue.

10. Kit d'un dispositif de cathéter médicinal, comprenant
un cathéter, et
une composition de verrouillage de cathéter étant un gel thixotrope.
